(19) Europäisches Patentamt
European Patent Office
Office européen des brevets

(11) **EP 1 903 986 B1**

(12) **EUROPÄISCHE PATENTSCHRIFT**

(45) Veröffentlichungstag und Bekanntmachung des Hinweises auf die Patenterteilung:
**15.02.2012 Patentblatt 2012/07**

(21) Anmeldenummer: **06743184.1**

(22) Anmeldetag: **22.06.2006**

(51) Int Cl.:
*A61F 2/16* $^{(2006.01)}$ *G02C 7/02* $^{(2006.01)}$

(86) Internationale Anmeldenummer:
**PCT/EP2006/006041**

(87) Internationale Veröffentlichungsnummer:
**WO 2006/136424 (28.12.2006 Gazette 2006/52)**

(54) **ASTIGMATISCHE INTRAOKULARLINSE**

ASTIGMATIC INTRAOCULAR LENS

LENTILLE INTRAOCULAIRE ASTIGMATIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.06.2005 DE 102005028933**

(43) Veröffentlichungstag der Anmeldung:
**02.04.2008 Patentblatt 2008/14**

(73) Patentinhaber: **Carl Zeiss Meditec AG 07745 Jena (DE)**

(72) Erfinder:
• **FIALA, Werner A-1180 Wien (AT)**

• **KREINER, Christine 81545 München (DE)**

(74) Vertreter: **Nöth, Heinz et al Eisenführ, Speiser & Partner Postfach 31 02 60 80102 München (DE)**

(56) Entgegenhaltungen:
**EP-A2- 0 742 461 EP-A2- 0 949 529 WO-A-03/009053 WO-A2-2004/090611 US-A1- 2003 014 107**

**Beschreibung**

Die vorliegende Erfindung betrifft eine Intraokularlinse (IOL) zur Korrektur einer astigmatischen Fehlsichtigkeit.

Hintergrund der Erfindung

Linsen dienen allgemein zur Umwandlung einer Lichtwelle in eine andere Lichtwelle. Eine wesentliche Aufgabe der Linsenoptik ist es, eine sphärische Lichtwelle aus einem Gegenstandspunkt so umzuwandeln, dass die resultierende Welle ebenfalls sphärisch ist und somit der zum Gegenstandspunkt konjugierte Bildpunkt das Zentrum der resultierenden sphärischen Wellenfronten ist. Eine von einem Gegenstandspunkt einfallende Welle ist stets sphärisch; befindet sich der Gegenstandspunkt in (praktisch) unendlicher Entfernung, so ist die einfallende Welle plan bzw. eben. In der Folge werden auch einfallende ebene Wellen unter dem Oberbegriff "einfallende sphärische Welle" subsummiert.

Linsensysteme zur Abbildung eines Gegenstandspunktes in einen konjugierten Bildpunkt können Linsen oder brechende Flächen enthalten, die keine Rotationssymmetrie aufweisen. Eine einfallende sphärische Welle wird von solchen Linsen bzw. Flächen in eine Welle umgewandelt, deren Wellenfronten keine Rotationssymmetrie aufweisen; die Wellenfronten sind "werzerrt". Solche Wellenfronten sind dann mittels sogenannter "astigmatischer" Linsen oder brechender Flächen, die ihrerseits keine Rotationssymmetrie aufweisen, in Wellen umzuwandeln, deren Wellenfronten wiederum Rotationssymmetrie besitzen.

Ein bekanntes Beispiel einer brechenden Fläche, die eine einfallende sphärische Welle in eine Welle umwandelt, deren Wellenfronten keine Rotationssymmetrie aufweisen, ist die Oberfläche einer astigmatischen Hornhaut (Comea) oder einer astigmatischen Linse des Auges. Die verzerrten Wellenfronten sind dann im pseudo-phaken Auge durch astigmatische Linsen in Wellenfronten mit Rotationssymmetrie, in der Regel in sphärische Wellen, umzuwandeln. Zur Korrektur des Astigmatismus der Hornhaut bzw. des Auges, der im Wesentlichen auf eine nicht-sphärisch (z.B. torisch) geformte Hornhaut und/oder andere Anomalien des Auges, wie z.B. eine Augenlinse ohne Rotationssymmetrie bzw. eine Netzhautanomalie zurückzuführen ist, werden Kontaktlinsen, Intraokularlinsen und auch Brillengläser eingesetzt. Vorliegend werden nur Intraokularlinsen behandelt.

Bekannte Intraokularlinsen zur Korrektur einer astigmatischen Fehlsichtigkeit weisen eine zylindrisch oder torisch brechende Begrenzungsfläche und eine (sphärisch oder asphärisch) rotationssymmetrisch brechende Begrenzungsfläche auf.

Eine torisch brechende oder torische Fläche entsteht durch Rotation eines Kreisbogensegments oder eines Kreises um eine Achse, die den Mittelpunkt des Kreises nicht enthält. Ist der größte Abstand zwischen dem Kreis und der Rotationsachse kleiner als der Kreisradius, so wird die resultierende torische Fläche "tonnenförmig" genannt, ist dieser Abstand größer als der Kreisradius, so handelt es sich um eine "wurstförmige" torische Fläche (gemäß Handbuch für Augenoptik, Herausgeber: Carl Zeiss, Seite 23 (1977)). Im Sprachgebrauch werden "torisch" und "astigmatisch" häufig synonym verwendet. Nachfolgend wird durchgehend die Bezeichnungen "torisch" verwendet, auch wenn die so beschriebene Linsenoberfläche nicht im mathematischen Sinne torisch ist.

Durch die torische Linsenoberflläche wird erreicht, dass die Brechkraft der IOL in einem Meridian verschieden von der Brechkraft im anderen Meridian ist. Die beiden Meridiane stehen senkrecht zueinander. Der Unterschied der beiden Brechkräfte wird üblicherweise als Linsenzylinder bezeichnet, da die optische Wirkung solcher torischer IOL jener einer Kombination einer sphärischen Linse und einer Zylinderlinse entspricht. Eine Zylinderlinse besitzt für die Einfallsebene des Lichts, die die Zylinderachse enthält, die Brechkraft Null und in der zu dieser Einfallsebene senkrechten Ebene die maximale Brechkraft, die im Wesentlichen durch den Radius des Zylinders und den Brechungsindex der Zylinderlinse gegeben ist. Diese maximale Brechkraft der Zylinderlinse wird also kurz als "Linsenzylinder" (in Dioptrien) bezeichnet. Aus dem Gesagtem wir klar, dass die beiden Meridiane einer torischen Linse, in denen die Linsenbrechkraft einerseits den Maximalwert und andererseits den Minimalwert aufweist, -die sogenannten Hauptmeridiane - senkrecht aufeinander stehen und dass die Differenz aus maximaler Brechkraft und minimaler Brechkraft der Linsenzylinder der torischen Linse ist.

Anders gesagt besitzt die torische Fläche in den Hauptmeridianen unterschiedliche Krümmungsradien. In jeder zur Linsenachse senkrechten Ebene ist die Schnittlinie der (sphärisch oder asphärisch) rotationssymmetrisch brechenden Begrenzungsfläche ein Kreis und die Schnittlinie der torischen Fläche eine Ellipse. Die kleine Achse dieser Ellipse ist durch den kleineren Radius im zugehörigen Hauptmeridian, die große Ellipsenachse ist durch den größeren Krümmungsradius im anderen Hauptmeridian bestimmt. Aufgrund der elliptischen Form ist im Falle der Begrenzung der torischen Fläche durch eine Ebene die optisch wirksame Fläche für die größere der beiden Brechkräfte der Linse kleiner als für die kleinere der beiden Brechkräfte.

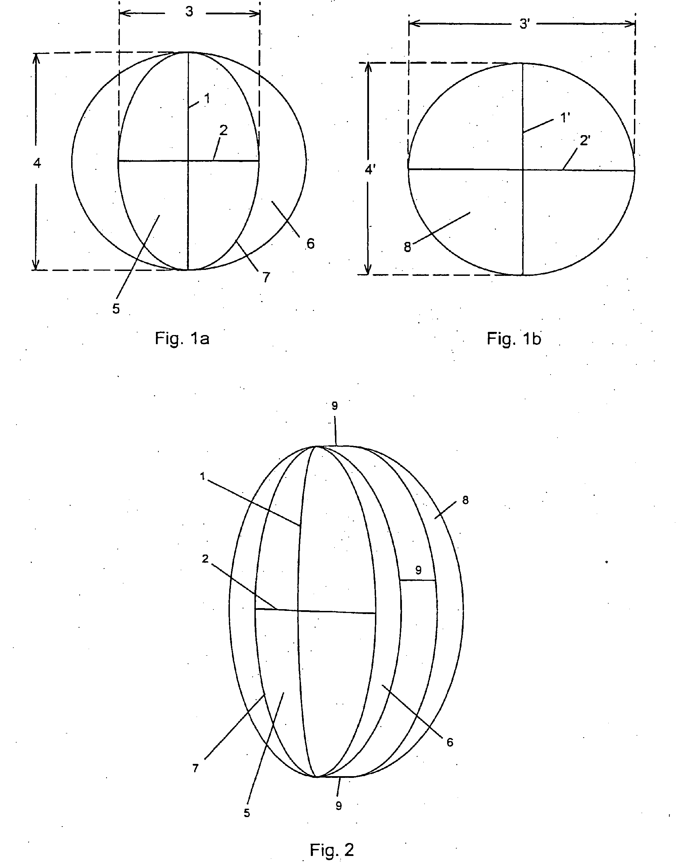

In Fig. 1a ist schematisch die Projektion der torischen Fläche einer herkömmlichen torischen IOL dargestellt. In dieser Figur ist 1 der Meridian mit der kleineren der beiden Brechkräfte, 2 der Meridian der größeren der beiden Brechkräfte. Der Radius R1 im Meridian 1 ist größer alls der Radius R2 im Meridian 2, d.h. R1>R2. Die auf eine ebene Fläche 6 projizierte Ellipse 7 der torischen brechenden Fläche 5 besitzt die kleinere Achse 3 und die größere Achse 4.

Die optisch wirksame Fläche ist auf den inneren Bereich der Ellipse gemäß Fig. 1a begrenzt. Die Projektion der anderen, nämlich der sphärischen, brechenden Fläche 8 der herkömmlichen torischen Linse ist in Fig. 1b dargestellt. Die Schnittlinie dieser brechenden Fläche mit einer Ebene ist ein Kreis, dessen "Achsenlängen" 3' und 4' entlang der beiden Meridiane 1' und 2' trivialerweise gleich sind.

In Fig. 2 ist eine schematische perspektivische Darstellung einer solchen Linse mit einer über den gesamten Umfang konstanten Randdicke 9 wiedergegeben.

Herkömmliche torische Linsen weisen in der Regel somit bei gegebener konstanter Randdicke eine optisch wirksame Fläche auf, die auf die Fläche einer Ellipse reduziert ist.

Ferner weisen herkömmliche torische Linsen zwei brechende Flächen auf, wobei die eine Fläche sphärisch ist und die andere Fläche torisch ist; die Schnittlinien in den Hauptmeridianen der torischen Linsenoberfläche werden aufgrund ihrer Rotationssymmetrie durch Kreisgleichungen bestimmt, wobei die Hauptmeridiane wie zuvor beschrieben, orthogonal zueinander angeordnet sind. Zur Umwandlung von Wellenfronten, die durch Brechung an astigmatischen brechenden Flächen wie der Comea entstehen, in sphärische Wellen, sind solche Linsen jedoch nur in seltenen Fällen gut geeignet.

Aus EP 0 949 529 A2 ist eine torische multifokale Linse, welche als Intraokularlinse verwendet werden kann, bekannt. Die Linse besitzt mehrere Sehkorrekturbereiche für Nahsicht und Fernsicht. Jeder Sehkorrekturbereich ist als torisch brechende Linsenoberfläche ausgebildet, wobei die beiden Hauptmeridiane in jedem Sehkorrekturbereich bestimmte Radien aufweisen, d.h. sphärisch gekrümmt sind. Sowohl auf der Linsenvorderseite als auch auf der Linsenrückseite können torisch brechende Oberflächenbereiche vorgesehen sein. Hierdurch kann eine relativ hohe optische Zylinderwirkung der Linse erreicht werden.

Aufgabe der Erfindung ist es, die optisch wirksame Fläche einer torischen (bzw. astigmatischen) IOL zu vergrößern und die Korrektureigenschaften bei astigmatischer Fehlsichtigkeit zu verbessern.

Gegenstand der Erfindung

Erfindungsgemäß wird diese Aufgabe durch die Merkmale des Patentanspruches 1 gelöst.

Die Schnittkurven der torisch brechenden Linsenoberfläche in zur Linsenachse parallelen. Ebenen werden in beiden Hauptmeridianen jeweils durch eine Asphäre beschrieben.

Bevorzugt sind dabei die Vorderfläche und die Rückfläche konvex geformt. In den seltenen Fällen einer negativen Linse sind beide Flächen konkav. Jedenfalls aber ist es vorteilhaft, wenn die Vorderfläche und die Rückfläche die gleiche Krümmungsrichtung und somit deren Brechung dasselbe Vorzeichen aufweisen. Besonders bevorzugt ist die zylindrische Wirkung in etwa gleichmäßig auf die Vorderfläche und die Rückfläche verteilt. Bei gleicher Verteilung - also identischer torischer Oberfläche auf der Vorseite und Rückseite der IOL - ist die optisch wirksame Fläche am größten. Selbstverständlich fallen auch ungleiche Verteilungen der Torizität unter den Gegenstand der Erfindung, bei denen eine Optimierung der Größe der optisch wirksamen Fläche hinter andere Abbildungseigenschaften zurücktritt.

Oberflächen, deren Schnittkurve in einem Hauptmeridian asphärisch sind, werden hierin ebenfalls als torische Linsenoberflächen bezeichnet. Solche Oberflächen entstehen durch Rotation nicht kreisbogenförmiger Kurven, denn bei torischen Flächen, die stets durch die Rotation einer Kurve entstehen, ist zumindest einer der beiden Meridiane kreisförmig. Obgleich Flächen, die nicht als Ergebnis einer Rotation einer Kurve dargestellt werden können, weil deren Schnittkurven in beiden Hauptmeridianen asphärisch sind, und die daher keine "torischen" Flächen im eigentlich mathematischen Sinn sind, werden auch diese hierin unter torisch subsummiert. Die Schnittlinien der zur Linsenachse parallelen Ebenen in den Hauptmeridianen solcher astigmatischer Flächen sind durch Gleichungen bestimmt, die für asphärische Kurven gelten. Hierauf wird später genauer eingegangen.

Ist die torische Oberfläche der IOL nicht auf solche beschränkt, die sich durch Rotation eines Kreisbogens erzeugen lässt, kann die Abbildungseigenschaft der erfindungsgemäßen IOL besser an die spezifische Form der durch Brechung einer einfallenden sphärischen Welle an einer astigmatischen Fläche des Auges bzw. Linse entstehenden Wellenfront angepasst werden

Die Intraokularlinse kann eine torisch brechende Linsenoberfläche mit zwei Hauptmeridianen aufweist, die einen Zwischenwinkel ungleich 90° einschließen. Dabei kann die gegenüberliegende Linsenoberfläche wiederum ebenfalls torisch, rotationssymmetrisch sphärisch oder rotationssymmetrisch asphärisch ausgebildet sein.

Brechende Flächen, bei denen der Meridian mit der größten Brechkraft mit dem Meridian mit der kleinsten Brechkraft einen Winkel ungleich 90° einschließt, werden wiederum streng mathematisch nicht als "torische" Flächen bezeichnet, hiernach dennoch unter diesem Begriff subsummiert.

Auch ein Winkel zwischen den Ebenen durch die Hauptmeridiane, der nicht auf den Wert von 90° beschränkt ist, dient dazu die Abbildungseigenschaft der erfindungsgemäßen IOL besser an die spezifische Form der durch Brechung einer einfallenden sphärischen Welle an einer astigmatischen Fläche des Auges bzw. Linse entstehenden Wellenfront anzupassen.

Auch sieht die Erfindung in einer vorteilhaften Ausführungsform vor, dass die Intraokularlinse die Merkmale einer torisch brechenden Vorderfläche und einer torisch brechenden Rückfläche mit denen wenigstens einer torisch brechenden Linsenoberfläche kombiniert, deren Schnittkurve oder Schnittkurven einer bzw. zweier zur Linsenachse paralleler Ebenen in einem bzw. beiden Hauptmeridianen durch Asphären beschrieben werden.

Ferner sieht die Erfindung in einer weiteren vorteilhaften Ausführungsform vor, dass die Intraokularlinse die Merkmale einer torisch brechenden Vorderfläche und einer torisch brechenden Rückfläche mit denen einer torisch brechenden Linsenoberfläche kombiniert, deren zwei Hauptmeridiane einen Zwischenwinkel ungleich 90° einschließen.

Weiterhin sieht die Erfindung in einer vorteilhaften Ausführungsform vor, dass die Intraokularlinse die Merkmale einer torisch brechenden Linsenoberfläche, deren Schnittkurve oder Schnittkurven einer bzw. zweier zur Linsenachse paralleler Ebenen in einem bzw. beiden Hauptmeridianen durch Asphären beschrieben werden, mit denen einer torisch brechenden Linsenoberfläche kombiniert, deren zwei Hauptmeridiane einen Zwischenwinkel ungleich 90° einschließen.

Schließlich sieht die Erfindung in einer weiteren vorteilhaften Ausführungsform vor, dass die Intraokularlinse die Merkmale einer torisch brechenden Vorderfläche und einer torisch brechenden Rückfläche mit denen wenigstens einer torisch brechenden Linsenoberfläche, deren Schnittkurve oder Schnittkurven einer bzw. zweier zur Linsenachse paralleler Ebenen in einem bzw. beiden Hauptmeridianen durch Asphären beschrieben werden, und denen einer torisch brechenden Linsenoberfläche kombiniert, deren zwei Hauptmeridiane einen Zwischenwinkel ungleich 90° einschließen.

Die erfindungsgemäße torische IOL (oder eigentlich IOL zu Korrektur einer astigmatischen Fehlsichtigkeit) kann mittels der genannten Merkmale sehr viel präziser auf die individuelle Fehlsichtigkeit durch eine astigmatische Fläche des zu behandelnden Auges eingestellt werden, so dass sie Wellenfronten, die durch die Brechung von Licht an dieser nicht-sphärischen astigmatischen Fläche (Cornea, Linse des Auges, etc.) gebildet werden, in im Wesentlichen sphärische Wellenfronten umwandelt. Anders gesagt, wird die erfindungsgemäße IOL, die von von einem Gegenstandspunkt ausgehenden und durch astigmatische brechende Flächen (Cornea, Linse des Auges, etc.) gebrochenen Strahlen derart gebrochen, dass dieser Gegenstandspunkt im Wesentlichen in genau einem Bildpunkt abgebildet wird. Die Abbildung des Gesamtsystems bestehend aus astigmatischen brechender Fläche des Auges und der erfindungsgemäßen IOL ist stigmatisch. An dieser Stelle wird darauf hingewiesen, dass die erfindungsgemäße IOL sowohl sog. "phake" als auch sog. "pseudophake" Intraokularlinsen umfasst, dass heißt sowohl solche die an Stelle der natürlichen Linse des Auges als auch zusätzlich zu dieser implantiert werden.

Weitere Merkmale und Vorteile der erfindungsgemäßen IOL ergeben sich aus den Unteransprüchen. Diese werden unter Bezugnahme auf die beigefügten Figuren in der nachfolgenden Beschreibung erläutert. Es zeigen:

Fig. 1    eine schematische Projektion der torischen Fläche einer torischen IOL gemäß dem Stand der Technik;

Fig. 2    eine schematische perspektivische Darstellung der bekannten IOL gemäß Fig. 1;

Fig. 3    eine schematische Projektion der Linsenfrontfläche (Fig. 3a) und der Linsenrückfläche (Fig. 3b) einer Ausführungsform der erfindungsgemäßen IOL auf eine Ebene;

Fig. 4    eine schematische Projektion der torischen Fläche eines Ausführungsbeispiels der erfindungsgemäßen IOL, mit einem (Zwischen-)Meridian der Funktion eines Winkels α;

Fig. 5    einen Graphen, in dem drei Funktionen für den Krümmungsradius der torischen Fläche des Ausführungsbeispiels gemäß Fig. 4 gegen den Winkel α aufgetragen sind, und

Fig. 6    einen Aufriss der Vorfläche Fig. 6a und einen Aufriss der zugehörigen Rückfläche Fig. 6b der torischen Fläche eines Ausführungsbeispiels der erfindungsgemäßen IOL, deren Hauptmeridian mit der kleinsten Brechkraft mit dem Hauptmeridian mit der größten Brechkraft einen Winkel χ ungleich 90° einschließt.

Wird bei einer torischen Linse eine torische Fläche, z.B. die Frontfläche, mit einer sphärischen Fläche, z.B. der Rückfläche, kombiniert, so ist die Projektion dieser torischen (Front)fläche auf eine Ebene eine Ellipse mit den Achsen 1 und 2 gemäß Fig.1a. Die Länge der großen Ellipsenachse 1 sei mit $a_m$ bezeichnet, die Länge der kleinen Ellipsenachse 2 sei mit $b_m$ bezeichnet. Weiterhin sei der Krümmungsradius der torischen Fläche im Hauptmeridian 1 mit $R1_m$ und der Krümmungsradius im Hauptmeridian 2 mit $R2_m$ bezeichnet. Der Radius der sphärischen (Rück)fläche der torischen Linse sei mit $R_s$ bezeichnet. Die Brechkraft D1 im Hauptmeridian 1 der Linse ist dann gegeben durch:

$$D1 = \frac{n_L - n_{imm}}{R1_m} + \frac{n_L - n_{imm}}{R_s} - \frac{t(n_L - n_{imm})^2}{n_L R1_m R_s} \qquad (1)$$

wobei:

$n_L$ Index der Linse
$n_{imm.}$ Index des die Linse umgebenden Mediums
$t.$ Mittendicke der Linse

sind. In Gleichung (1) haben Radien einen positiven Wert, wenn sie konvex für Licht sind, das auf die betrachtete Linsenfläche von außen einfällt bzw. einfallen würde. Bei einer bikonvexen Linse sind somit sowohl $R1_m$ als auch $R_s$ positiv.

In gleicher Weise ist die Brechkraft D2 im Hauptmeridian 2 der Linse gegeben durch:

$$D2 = \frac{n_L - n_{imm}}{R2_m} + \frac{n_L - n_{imm}}{R_s} - \frac{t(n_L - n_{imm})^2}{n_L R2_m R_s} \qquad (2)$$

Der Zylinder Z der torischen Linse ist in sehr guter Näherung gegeben durch

$$Z = D2 - D1 \approx \frac{n_L - n_{imm}}{R2_m} - \frac{n_L - n_{imm}}{R1_m}, \qquad (3)$$

da die Differenz der beiden dritten Glieder aus Gleichung (1) und (2) ausgesprochen klein ist. Da die dritten Glieder (die lediglich einen Korrekturfaktor für die Mittendicke der Linse darstellen) in den Gleichungen (1) und (2) gegenüber den anderen Gliedern klein sind, werden sie in den folgenden prinzipiellen Überlegungen nicht berücksichtigt.

Wird eine erfindungsgemäße Linse demgegenüber so gestaltet, dass sowohl die Frontfläche wie auch die Rückfläche torisch sind, dann sind bei gleicher zylindrischer Wirkung der Linse die Längen $b_b$ der Ellipsenachsen der beiden torischen Flächen größer als $b_m$, d.h. die wirksame optische Fläche ist im Vergleich zu einer herkömmlichen torischen Linse mit nur einer torischen und einer sphärischen brechenden Fläche größer. Als Beispiel sei angenommen, dass die zylindrische Wirkung der Linse zu gleichen Teilen auf die Vorder- und die Rückfläche aufgeteilt wird. Dann muss jede dieser Flächen den Zylinder Z/2 aufweisen. Der Radius im Hauptmeridian mit der kleineren Brechkraft sei $R1_b$, und ohne Beschränkung der Allgemeinheit sei angenommen, dass $R1_b = R1_m$ sei. Dann ist der Radius $R2_b$ im Hauptmeridian mit der größeren Brechkraft durch folgende Gleichung in sehr guter Näherung bestimmt:

$$\frac{Z}{2} \approx \frac{n_L - n_{imm}}{R2_b} - \frac{n_L - n_{imm}}{R1_m} \qquad (4)$$

Kombinieren von Gleichungen (3) und (4) und Lösen nach $R2_b$ ergibt:

$$R2_b = \frac{2R1_m R2_m}{R1_m + R2_m} > R2_m, \qquad (5)$$

da $R2_m < R1_m$ ist.

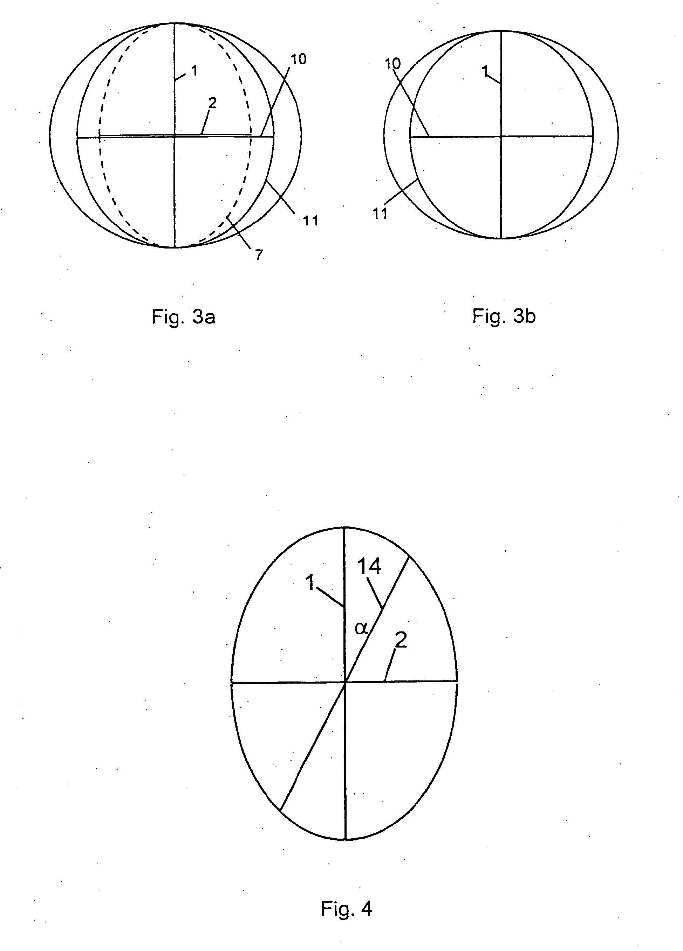

In Fig. 3a und 3b sind schematisch die Verhältnisse dargestellt. Dabei ist Fig. 3a die Projektion der Linsenfrontfläche auf eine Ebene und Fig. 3b die Projektion der Rückfläche der erfindungsgemäßen Linse auf eine Ebene. In dieser Figur sind 1 der Hauptmeridian der kleineren Brechkraft, 2 der Hauptmeridian der größeren Brechkraft einer torischen Linse, die als zweite brechende Fläche eine sphärische Fläche besitzt, und 10 ist der Hauptmeridian einer torischen Linse die zwei gleichartige, spiegelgleiche torisch brechende Flächen besitzt; beide Linsen weisen denselben Zylinder auf. Die große Ellipsenachse im Hauptmeridian 1 der kleineren Brechkraft ist im Fall der Linse mit nur einer torisch brechenden Fläche $a_m$ sowie im Fall der Linse mit zwei torischen Flächen $a_b$. Diese Ellipsenachse ist hier in beiden Fällen identisch, d.h. es gilt $a_b = a_m$. Die kleine Ellipsenachse im Hauptmeridian 2 der größeren Brechkraft der Linse mit nur einer torischen Fläche ist $b_m$, die kleine Ellipsenachse im Hauptmeridian 10 der größeren Brechkraft der Linse mit zwei torischen Flächen ist $b_b$. Es ist ersichtlich, dass die wirksame optische Fläche der Linse mit zwei torischen Flächen größer ist als jene mit nur einer torischen Fläche und einer sphärischen Fläche.

Die Aufteilung des Zylinders zu gleichen Teilen auf Vorder- und Rückfläche einer erfindungsgemäßen Linse ist willkürlich; selbstverständlich sind auch andere Aufteilungen dieses Zylinders möglich.

Zur Veranschaulichung seien die Ergebnisse für Linsen mit der kleineren Brechkraft von 10 Dioptrien und einem Zylinder von +6 Dioptrien gebracht. Die Linse sei aus einem Material mit Brechungsindex $n_L$ = 1.49 gefertigt und befinde sich in einem Medium mit Brechungsindex $n_{imm}$ = 1.336. Die große Ellipsenachse (für die kleinere Brechkraft von 10 Dioptrien) sei 10 mm. Dann erhält man für die kleine Ellipsenachse für eine Linse mit nur einer torischen und einer sphärischen brechenden Fläche den Wert von 6.03 mm; für die kleine Ellipsenachse der Linse mit zwei spiegelgleichen torischen Flächen erhält man den Wert von 7.9 mm.

Die obigen Überlegungen gelten für torische Linsen, deren Schnittkurven in den Hauptmeridianen als Sonderfall durch Kreisgleichungen gegeben sind.

Für die Umwandlung von Wellenfronten, die durch Brechung einer ebenen Welle an einer astigmatischen bzw. torischen Fläche entstehen, in sphärische Wellen, können astigmatische Linsen, deren Schnittkurven in den Hauptmeridianen durch Asphärengleichungen gegeben sind, erforderlich sein. Erfindungsgemäß weisen deshalb astigmatische Linsen in den Hauptmeridianen Kurven auf, die durch Asphärengleichungen gegeben sind. Bei solchen erfindungsgemäßen Linsen sind die Gleichungen der Kurven in den Hauptmeridianen der brechenden Fläche bzw. Flächen durch die Scheitelgleichungen von Asphären gegeben. Die Form dieser Scheitelgleichungen ist

$$y = \sqrt{2Rx - (1 + asp)x^2} \qquad (6)$$

wobei

$R$      *Radius bzw. Parameter im Apex der Linse*
$asp$    *Asphärizität der Asphäre*

sind. Der Radius $R$ ist entsprechend durch $R1_m$ oder $R2_m$ oder $R1_b$ oder $R2_b$ gegeben, da für achsnahe Lichtstrahlen, d.h. innerhalb des Bereiches der Gaußschen Optik, die Brechungsgesetze an Linsen für sphärische und asphärische Flächen des gleichen Radius bzw. Parameters praktisch gleich sind.

Die Asphärizitäten in den beiden Hauptmeridianen können verschieden sein. Bezeichnet man mit **asp1** die Asphärizität im Hauptmeridian 1 und mit **asp2** die Asphärizität im Hauptmeridian 2, so gilt entweder:

$$asp1 = asp2 \qquad (7)$$

oder

$$asp1 \neq asp2 \qquad (7')$$

Die Werte für die geeigneten Werte für **asp1** und **asp2** hängen von der Form der Welle ab, die durch Brechung einer planen Welle an einer astigmatischen brechenden Fläche entsteht.

Für bestimmte Anwendungsfälle kann es erforderlich sein, in den Hauptmeridianen Schnittkurven mit der brechenden Fläche erfindungsgemäßer Linsen vorzusehen, die nicht durch die Scheitelgleichung einer einfachen Asphäre gemäß Gleichung (6), d.h. durch die Gleichung eines Kegelschnittes, dargestellt werden können. Dann ist es sinnvoll, eine andere Darstellungsform für diese Schnittkurve zu wählen, die man durch Lösen der Gleichung (6) und durch Addition einer Potenzreihe erhält:

$$x = \frac{y^2 / R}{1 + \sqrt{1 - (1 + asp)y^2 / R^2}} + \sum_j c_j y^j \tag{8}$$

wobei $c_j$ Polynomialkoeffizienten sind.

Wird eine Darstellungsform der Asphäre gemäß-Gleichung (8) gewählt, so sind in jedem der Hauptmeridiane neben der Asphärizität auch die Werte für die Koeffizienten $c_j$ zu bestimmen; in der Regel werden wegen der üblichen Monotonie der Kurve gemäß Gleichung (8) nur geradzahlige Glieder in Gleichung (8) berücksichtigt.

Die für erfindungsgemäße Linsen mit Asphärengleichungen in den Schnittkurven der Hauptmeridiane der brechenden Fläche bzw. Flächen gemäß Gleichung (7) geltenden allgemeinen Überlegungen gelten auch mutatis mutandis für erfindungsgemäße Linsen mit Asphärengleichungen in den entsprechenden Schnittkurven gemäß Gleichung (8).

Allgemein hängt die Wahl der verschiedenen Parameter $R$ und $asp$ in den Meridianen der einen oder der beiden torischen bzw. astigmatischen Flächen bzw. der sphärischen Fläche einer torischen Linse von der einfallenden Welle ab, die durch diese Linse in eine sphärische Welle umgewandelt werden soll. Die Bestimmung der Werte für die beiden Radien bzw. Parameter und den beiden Asphärizitäten in den Hauptmeridianen der Linse kann durch rechnergestützte iterative Verfahren erfolgen, bei denen sämtliche betrachtete Lichtstrahlen an den vorhandenen Flächen gebrochen werden. Solche Verfahren erfordern die analytische dreidimensionale Darstellung sämtlicher brechender Flächen im Linsensystem, die Berechnung der Normalvektoren in beliebigen Positionen auf diesen Flächen (Gradientenbildung) und das dreidimensionale Brechungsgesetz in Vektorform. Für den Fachmahn sind solche Verfahren einsichtig; sie stellen keinen Gegenstand der gegenständlichen Erfindung dar. Weiterhin können allgemeine Richtlinien zur geeigneten Wahl dieser Parameter der Patentoffenlegungsschrift (W. Fiala et al, PCT/EP2004/006074 published as WO 2004/108017) entnommen werden.

Eine erfindungsgemäße Linse weist somit in den Hauptmeridianen der brechenden Fläche bzw. Flächen Kurven auf, die analytisch durch die Scheitelgleichungen von Asphären gemäß Gleichung (6) bzw. Gleichung (8) dargestellt werden können. D.h. die Schnittkurven von achsparallelen Ebenen in den Hauptmeridianen sind im Fall der Darstellung gemäß Gleichung (6) Asphären mit einem Parameter bzw. Radius $R$ und einer Asphärizität $asp$ und im Fall der Darstellung gemäß Gleichung (8) zusätzlich mit den Koeffizienten $c_j$.

Die Schnittkurven von achsparallelen Ebenen in den Meridianen zwischen den Hauptmeridianen können als Funktion der Parameter bzw. Radien und der Asphärizitäten in den Hauptmeridiane und des Winkels zwischen dem betrachteten Meridian und einem der Hauptmeridiane dargestellt werden. In Fig. 4 sind die Verhältnisse dargestellt. Der Hauptmeridian mit der kleinsten Brechkraft ist wieder mit 1 bezeichnet, der Hauptmeridian mit der größten Brechkraft ist mit 2 bezeichnet. Ein Meridian 14 bilde einen Winkel $\alpha$ mit dem Hauptmeridian 1. Der Radius bzw. Parameter in diesem Meridian sei mit $R_\alpha$ bezeichnet.

Für den Radius bzw. Parameter $R_\alpha$ gilt dann allgemein:

$$R_\alpha = f(\alpha) \tag{9}$$

mit den Randbedingungen:

$$
\begin{aligned}
&f(0) = f(180°) = R1 \\
&f(90°) = f(270°) = R2 \\
&f'(\alpha) < 0 \ \textit{für} \ 0 < \alpha < 90° \ \textit{und} \ \ 180° < \alpha < 270° \\
&f'(\alpha) > 0 \ \textit{für} \ 90° < \alpha < 180° \ \textit{und} \ \ 270° < \alpha < 360°
\end{aligned}
\tag{10}
$$

d.h., dass wegen R1>R2 diese Funktion zwischen 0° und 90° und 180° und 270° Grad monoton sinkend und zwischen 90° und 180° bzw. 270° und 360° monoton steigend sein muss. Es ist einsichtig, dass für $f(\alpha)$ eine Vielzahl von Funktionen zur Auswahl steht. Die am besten geeignete Funktion $f(\alpha)$ kann nur in Kenntnis der auf die Linse einfallenden Wellenfront bestimmt werden, die durch die erfindungsgemäße Linse in eine sphärische Wellenfront umgewandelt werden soll. Zur Bestimmung der optimalen Funktion $f(\alpha)$ sind in der Regel verschiedene mathematische Ansätze für $f(\alpha)$ zu testen.

Lediglich als Beispiele gültiger Ansätze für die Funktion $f(\alpha)$ seien genannt:

$$R_\alpha = f_1(\alpha) = R1 \times \cos^2 \alpha + R2 \times \sin^2 \alpha \qquad (11)$$

oder

$$R_\alpha = f_2(\alpha) = \sqrt{R1^2 \times \cos^2 \alpha + R2^2 \times \sin^2 \alpha} \qquad (12)$$

oder

$$R_\alpha = f_3(\alpha) = \frac{1}{\dfrac{1}{R1}\cos^2 \alpha + \dfrac{1}{R2}\sin^2 \alpha} \qquad (13)$$

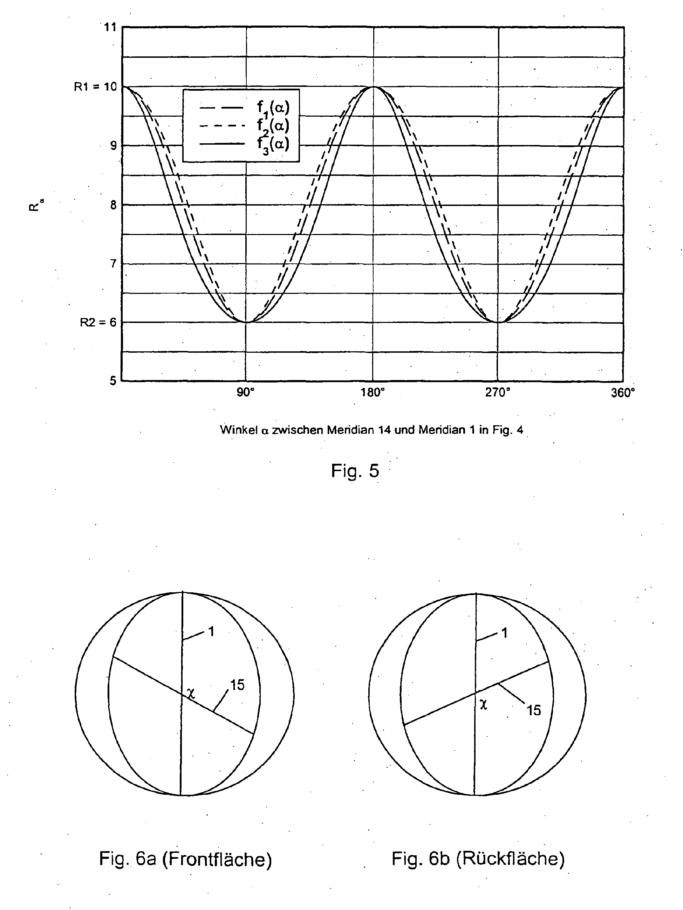

In Fig. 5 sind die obigen drei Funktionen für die Werte R1 = 10 und R2 = 6 beispielhaft für gültige Ansätze von $f(\alpha)$ dargestellt. Unzählige andere Funktionenformen für $f(\alpha)$ sind möglich.

Die Asphärizitäten in den Hauptmeridianen 1 und 2 sind meist verschieden, können aber auch gleich sein. Jedenfalls kann der Wert für die Asphärizität $asp_\alpha$ im Meridian 14 wieder als Funktion der beiden Aspärizitäten asp1 und asp2 in den Hauptmeridianen und des Winkels $\alpha$ angesetzt werden. Dabei können gleichartige Funktionen wie die soeben erwähnten $f(\alpha)$ oder andere Funktionen $g(\alpha)$ zum Einsatz kommen.

Bezeichnen wir generell die Funktion für den Parameter $R_\alpha$ mit $f(\alpha, R1, R2)$ und die Funktion für die Aspärizität $asp_\alpha$ der astigmatischen Linsenfläche mit $g(\alpha, asp1, asp2)$ dann lässt sich die astigmatische Fläche (ohne Glieder höherer Ordnung) wie folgt darstellen:

$$y^2 + z^2 = 2R_\alpha x - (1 + asp_\alpha)x^2 \qquad (14)$$

Gleichung (14) kann auch dargestellt werden als:

$$F(x, y, z) = x^2 + y^2 + z^2 - 2R_\alpha x + asp_\alpha x^2 = 0 \qquad (14')$$

wobei:

$$R_\alpha = f(\alpha, R1, R2)\ und$$
$$asp_\alpha = g(\alpha, asp1, asp2)\ ist. \qquad (15)$$

Liegt der Hauptmeridian in der xy-Ebene mit der x-Achse als Linsenachse, dann kann $\alpha$ dargestellt werden als

8

$$\alpha = \arctan(z/y) \qquad\qquad (16)$$

und durch Einsetzen von Gleichung (16) in Gleichung (14') erhält man die Darstellung der astigmatischen brechenden Fläche einer erfindungsgemäßen Linse in cartesischen Koordinaten.

Als Beispiel wird die Gleichung einer astigmatischen Fläche angeführt, bei der sowohl der Parameter $R_\alpha$ als auch die Asphärizität $asp_\alpha$ durch Funktionen des Typs $f_1(\alpha)$ dargestellt werden. Weiters sei die Richtung des Hauptmeridians 1 die y-Richtung und die Richtung des Hauptmeridians 2 die z-Richtung. Das heißt:

$$R1 = R_{xy} \quad und \quad asp1 = asp_{xy}$$

sowie

$$R2 = R_{xz} \quad und \quad asp2 = asp_{xz}$$

Damit erhält man für die astigmatische Fläche der erfindungsgemäßen Linse den Ausdruck

$$F(x,y,z) = x^2 + y^2 + z^2 + \frac{y^2}{y^2+z^2} asp_{xy} x^2 + \frac{z^2}{y^2+z^2} asp_{xz} x^2 -$$
$$- 2\frac{y^2}{y^2+z^2} R_{xy} x - 2\frac{z^2}{y^2+z^2} R_{xz} x = 0 \qquad (17)$$

Schließlich sei noch der Fall behandelt, dass die Hauptmeridiane einer astigmatischen brechenden Fläche nicht orthogonal zueinander stehen.

In Fig. 6a ist die Frontansicht (Aufriss) der astigmatischen Fläche einer astigmatischen Linse dargestellt, in Fig. 6b der Aufriss der zugehörigen Rückfläche dieser astigmatischen Linse.

Der Winkel zwischen den Hauptmeridianen 1 und 15 (Fig. 6a bzw. 6b) ist also nicht durch 90° sondern durch einen Winkel $\chi$ gegeben. Die Parameter in den Hauptmeridianen seien wiederum R1 und asp1 bzw. R2 und asp2. Dann können für Meridiane zwischen den nicht-orthogonalen Hauptmeridianen die Parameter $R_\alpha$ und $asp_\alpha$ in analoger Weise, wie oben gezeigt, dargestellt werden. In den Gleichungen (15) ist allerdings das Winkelargument $\alpha$ durch $\alpha'$ zu ersetzen, wobei der Zusammenhang zwischen diesen Werten durch folgende Beziehungen gegeben ist:

$$\alpha' = 90\frac{\alpha}{\chi} \qquad für \quad 0 \leq \alpha \leq \chi$$
$$\alpha' = 90\frac{180 - 2\chi + \alpha}{180 - \chi} \qquad für \quad \chi \leq \alpha \leq 180$$
$$\alpha' = 180 + 90\frac{\alpha - 180}{\chi} \qquad für \quad 180 \leq \alpha \leq 180 + \chi \qquad (18)$$
$$\alpha' = 180 + 90\frac{\alpha - 2\chi}{180 - \chi} \qquad für \quad 180 + \chi \leq \alpha \leq 360$$

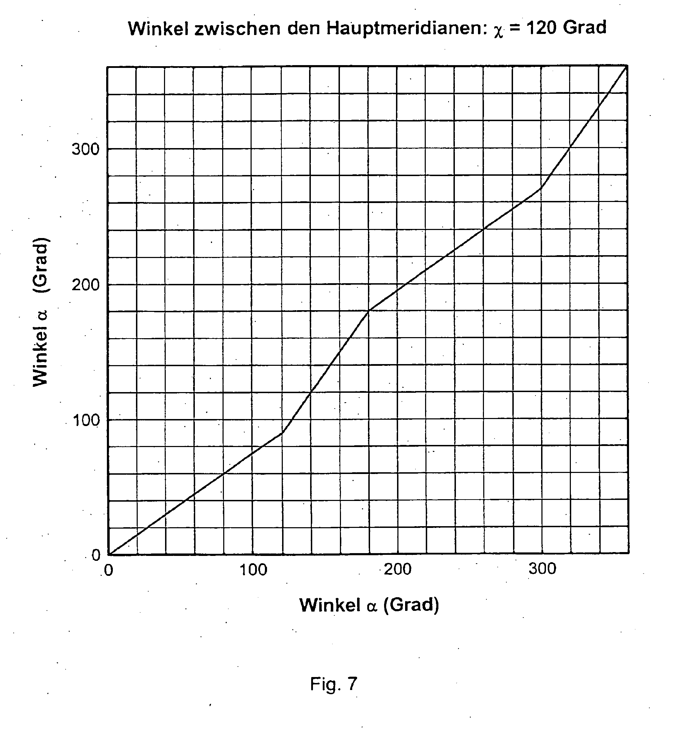

In Fig. 7 ist der Zusammenhang zwischen $\alpha$ und $\alpha'$ für den Fall eines Winkels $\chi$ = 120° dargestellt.

Die Zusammenhänge gemäß Gleichung (18) sind linear. Selbstverständlich kann der Zusammenhang zwischen $\alpha$ und $\alpha'$ durch andere, nichtlineare Funktionen $\alpha' = h(\alpha)$ hergestellt werden. Von der Funktion $h(\alpha)$ ist allerdings steigende Monotonie mit steigendem Wert für $\alpha$ zu fordern. Auch muss $h(\alpha)$ den Wert $\alpha'=90°$ für $\alpha = \chi$ ergeben, bzw. allgemein die Werte $\alpha'$ = 0, 90, 180, 270 und 360° für Winkel $\alpha$, die mit den Winkeln der Hauptmeridiane $(0,\chi,180°,180° + \chi,360°)$ zusammenfallen.

Erfindungsgemäße Linsen mit astigmatischen brechenden Flächen der beschriebenen Art sind zur Umwandlung von Wellenfronten, die durch Brechung an nicht-rotationssymmetrischen brechenden Flächen entstehen, in sphärische Wellenfronten wesentlich besser geeignet als herkömmliche torische Linsen mit einer torischen und einer sphärischen brechenden Fläche.

**Patentansprüche**

1. Intraokularlinse mit wenigstens einer torisch brechenden Linsenoberfläche zur Korrektur einer astigmatischen Fehlsichtigkeit sowohl an der Linsenvorderseite als auch an der Linsenrückseite, **dadurch gekennzeichnet, dass** die Schnittkurven der torisch brechenden Linsenoberfläche in beiden Hauptmeridianen in zur Linsenachse parallelen Ebenen jeweils durch eine Asphäre gemäß Gleichung

$$y = \sqrt{2Rx - (1 + asp)x^2}$$

oder

$$x = \frac{y^2/R}{1 + \sqrt{1 - (1 + asp)y^2/R^2}} + \sum_j c_j y^j$$

beschrieben sind, wobei

R Radius bzw. Parameter im Apex der Linse,
asp Asphärizität der Asphäre, und
$c_j$ Polynomialkoeffizienten sind.

2. Intraokularlinse nach Anspruch 1, **dadurch gekennzeichnet, dass** die Vorderfläche und die Rückfläche konvex geformt sind.

3. Intraokularlinse nach Ansprüch 1 oder 2, **dadurch gekennzeichnet, dass** die zylindrische Wirkung in etwa gleichmäßig auf die Vorderfläche und die Rückfläche verteilt ist.

4. Intraokularlinse nach einem der Ansprüche 1 bis 3, **dadurch gekennzeichnet, dass** die Intraokularlinse eine torisch brechende Linsenoberfläche mit zwei Hauptmeridianen aufweist, die einen Zwischenwinkel ungleich 90° einschließen.

**Claims**

1. An intraocular lens with at least one torically refractive lens surface for correcting astigmatism both on the front side of the lens and on the rear side of the lens, **characterised in that** the section curves of the torically refractive lens surface are described in both principal meridians in planes parallel to the lens axis in each case by an asphere in accordance with the equation

$$y = \sqrt{2Rx - (1 + asp)x^2}$$

or

$$x = \frac{y^2 / R}{1 + \sqrt{1 - (1 + asp)y^2 / R^2}} + \sum_j c_j y^j$$

with

R being the radius or parameter in the apex of the lens,
asp the asphericity of the asphere, and
$c_j$ polynomial coefficients.

**2.** An intraocular lens according to Claim 1, **characterised in that** the front surface and the rear surface are of convex shaped.

**3.** An intraocular lens according to Claim 1 or 2, **characterised in that** the cylindrical effect is distributed approximately evenly on the front surface and the rear surface.

**4.** An intraocular lens according to one of Claims 1 to 3, **characterised in that** the intraocular lens has a torically refractive lens surface with two principal meridians which enclose an intermediate angle which is not equal to 90°.

**Revendications**

**1.** Lentille intraoculaire ayant au moins une surface de lentille à réfringence torique pour la correction d'une amétropie astigmatique à la fois sur la face avant et sur la face arrière de la lentille, **caractérisée par** des courbes de coupe de la surface de la lentille à réfringence torique dans deux méridians principaux dans des plans parallèles à l'axe de la lentille décrits respectivement par une non-sphère d'équation

$$y = \sqrt{2Rx - (1 + asp)x^2}$$

ou

$$x = \frac{y^2 / R}{1 + \sqrt{1 - (1 + asp)y^2 / R^2}} + \sum_j c_j y^j$$

dans lesquelles
R rayon ou paramètre au sommet de la lentille,
asp asphéricité de la non-sphère, et
$c_j$ coefficients polynomiaux.

**2.** Lentille intraoculaire suivant la revendication 1, **caractérisé en ce que** la face avant et la face arrière sont convexes.

**3.** Lentille intraoculaire suivant la revendication 1 ou 2, **caractérisé en ce que** l'effet cylindrique est réparti à peu près uniformément sur la face avant et sur la face arrière.

4. Lentille intraoculaire suivant l'une des revendications 1 à 3, **caractérisé en ce que** la lentille intraoculaire a une surface de lentille à réfringence torique ayant deux méridians principaux qui font un angle entre eux différent de 90°.

Fig. 1a

Fig. 1b

Fig. 2

Fig. 3a

Fig. 3b

Fig. 4

Winkel α zwischen Meridian 14 und Meridian 1 in Fig. 4

Fig. 5

Fig. 6a (Frontfläche)

Fig. 6b (Rückfläche)

Winkel zwischen den Hauptmeridianen: χ = 120 Grad

Fig. 7

**IN DER BESCHREIBUNG AUFGEFÜHRTE DOKUMENTE**

**In der Beschreibung aufgeführte Patentdokumente**

- EP 0949529 A2
- EP 2004006074 W, W. Fiala
- WO 2004108017 A

**In der Beschreibung aufgeführte Nicht-Patentliteratur**

- Handbuch für Augenoptik. 1977, 23